# EUROPEAN PATENT SPECIFICATION

(11) **EP 4 543 325 B1**
(45) Date of publication and mention of the grant of the patent: **02.09.2026**
(21) Application number: 23735656.3
(22) Date of filing: 23.06.2023
(51) Int. Cl.: A61B 17/221, A61B 17/22, A61B 17/00, A61M 25/00

(54) **CATHETER TIP EXPANDABLE IN COMPRESSION**
KATHETERSPITZE, DIE BEI KOMPRESSION EXPANDIERBAR IST
POINTE DE CATHÉTER EXTENSIBLE EN COMPRESSION

(30) Priority: 24.06.2022 US 202217849323
(43) Date of publication of application: 30.04.2025
(73) Proprietor: Neuravi Limited, Galway H91 K5YD (IE)
(72) Inventor: KEATING, Karl, Galway, H91 K5YD (IE); VALE, David, Galway, H91 K5YD (IE)
(74) Representative: Carpmaels & Ransford LLP
(86) International application number: PCT/EP2023/067159
(87) International publication number: WO 2023/247771

(56) References cited:
- EP-A1- 3 875 047
- WO-A1-2022/074423
- WO-A1-2022/123565
- WO-A2-2022/020366
- US-A1- 2005 038 447
- US-A1- 2010 036 312
- US-A1- 2011 160 763
- US-A1- 2012 165 858

## Description

### FIELD

The present invention generally relates to devices for removing acute blockages from blood vessels during intravascular medical treatments. More specifically, the present invention relates to retrieval catheters with expandable tips into which an object or objects can be retrieved.

### BACKGROUND

Clot retrieval aspiration catheters and devices are used in mechanical thrombectomy for endovascular intervention, often in cases where patients are suffering from conditions such as acute ischemic stroke (AIS), myocardial infarction (MI), and pulmonary embolism (PE). Accessing the neurovascular bed in particular is challenging with conventional technology, as the target vessels are small in diameter, remote relative to the site of insertion, and highly tortuous. Traditional devices are often either too large in profile, lack the deliverability and flexibility needed to navigate particularly tortuous vessels, or are ineffective at removing a clot when delivered to the target site.

Many existing designs for aspiration retrieval catheters are often restricted to, for example, inner diameters of 6Fr (1 French is 1/3 Millimeters) or between approximately 1.7272 - 1.8796 mm (0.068 - 0.074 inches). Larger sizes require a larger guide or sheath to be used, which then necessitates a larger femoral access hole to close. Most physicians would prefer to use an 8Fr guide / 6Fr sheath combination, and few would be comfortable going beyond a 9Fr guide / 7Fr sheath combination. This means that once at the target site, a clot can often be larger in size than the inner diameter of the aspiration catheter and must otherwise be immediately compressed to enter the catheter mouth. This compression can lead to bunching up and subsequent shearing of the clot during retrieval. Firm, fibrin-rich clots can also become lodged in the fixed-mouth tip of these catheters making them more difficult to extract. This lodging can also result in shearing where softer portions break away from firmer regions of the clot.

Small diameters and fixed tip sizes are also less efficient at directing the aspiration necessary to remove blood and thrombus material during the procedure. Fixed tip sizes can cause a clot to shear or break apart as the clot enters the tip opening. The suction must be strong enough such that any fragmentation that may occur as a result of aspiration or the use of a mechanical thrombectomy device can be held stationary so that fragments cannot migrate and occlude distal vessels. However, when aspirating with a fixed-mouth catheter, a significant portion of the aspiration flow ends up coming from vessel fluid proximal to the tip of the catheter, where there is no clot, because the diameter of the funnel catheter is smaller than that of the vessel. This significantly reduces aspiration efficiency, lowering the success rate of clot removal.

Any catheter design attempting to overcome these challenges with an expanding distal tip or structure would need to have the strength to extract the clot and exert a steady radial force in the expanded state. The same structure would also need to be sufficiently flexible and elastic to survive the severe mechanical strains imparted when navigating tortuous vasculature when in a collapsed state.

As a result, there remains a need for improved catheter designs attempting to overcome the above-mentioned design challenges. The present designs are aimed at providing an improved retrieval catheter with an expansile tip section and methods for using such a catheter capable of improved performance.

WO 2022/020366 A2 describes a clot aspiration system intended for removing clot from a blood vessel including an aspiration assembly which will have two or more of the following components: an aspiration catheter, an inner catheter, an intermediate catheter, and an outer catheter, the latter typically being a guiding or other sheath. A transition structure is coupled to a distal end of the aspiration assembly to cover or fill an open distal end of one or more of the components of the aspiration assembly. The transition structure may be configured to facilitate introduction of the aspiration catheter into the patient's vasculature and/or advancement of the aspiration catheter through the vasculature to a target site, such as a cerebral target site which may be occluded with clot, thrombus, or other occlusive material.

US 2010/036312 A1 describes an apparatus for removing material within a body lumen that includes a catheter including a proximal end, a distal end for introduction into a body lumen, and an aspiration lumen extending therebetween; a guide member extending from the distal end and terminating in a distal tip, the guide member comprising a track adjacent a track lumen extending from the distal tip into the aspiration lumen; and an obstruction clearing device deployable from the guide member and retractable along the track. In addition or alternatively, the apparatus includes a cutting head reciprocal within the aspiration lumen adjacent the distal end for macerating material being aspirated into the aspiration lumen.

### SUMMARY

The catheter tips according to the invention are defined in independent claims 1 and 9. Embodiments are defined in the dependent claims.

It is an object of the disclosed designs to provide device to meet the above-stated needs. The designs can be for a clot retrieval catheter capable of removing a clot from cerebral arteries in patients suffering from AIS, from coronary native or graft vessels in patients suffering from MI, and from pulmonary arteries in patients suffering from PE and from other peripheral arterial and venous vessels in which a clot is causing an occlusion.

One example of the present disclosure provides a catheter tip. The catheter tip can include a support frame that includes a longitudinal axis, a collapsed configuration, an expanded deployed configuration, and a plurality of interconnected struts. The plurality of interconnected struts can define an axial series of expansion cells and can be joined at opposing pairs of x-connectors spaced 180 degrees apart about the longitudinal axis. Each opposing pair of x-connectors can be rotated 90 degrees about the longitudinal axis with respect to an adjacent opposing pair of x-connectors. The support frame can further include a collapsed inner diameter in the collapsed delivery configuration and a larger expanded inner diameter in the expanded deployed configuration when the support frame is placed in compression.

The catheter tip can include an offset mouth strut at the distal end of the support frame, at least a portion of the offset mouth strut residing in a plane forming an acute angle with respect to the longitudinal axis.

The support frame can include a proximal collar at a proximal end. The proximal collar can include a ring member circumferentially divided by at least one seam.

The support frame can expand from the collapsed inner diameter to the expanded inner diameter when impinged by an ingested clot.

The support frame can be configured to heat set to have the expanded inner diameter greater than the collapsed inner diameter.

The support frame can include a shape memory alloy with an Austenite finish temperature less than approximately 30 degrees Celsius.

The support frame can include an axial length that is less in the expanded deployed configuration than in the collapsed delivery configuration.

The support frame can include a maximum outer diameter in the expanded deployed configuration less than an inner diameter of a target vessel at a treatment site.

When in the collapsed delivery configuration, a distal end of the support frame can include a substantially circular cross section with a center substantially coincident with the longitudinal axis.

The interconnected struts of the catheter tip can include a curvilinear profile.

The axial series of expansion cells can include opposing pairs of cells spaced 180 degrees apart about the longitudinal axis, and each opposing pair of cells can be rotated 90 degrees about the longitudinal axis with respect to the adjacent pair of opposing cells.

The axial series of expansion cells can shorten longitudinally when the support frame is placed in compression.

Another example of the present disclosure provides another catheter tip. The catheter tip can include a support frame that includes a longitudinal axis, a collapsed delivery configuration, an expanded deployed configuration, and an axial series of hoop ribs extending in planes offset from the longitudinal axis. The hoop ribs can include a curvilinear profile, a non-planar cross section, and a distally unconnected peak. The support frame can include a mouth that has a larger expanded inner diameter when the support frame is placed in compression.

The support frame can include a larger expanded inner diameter when impinged radially by an ingested clot in the expanded deployed configuration and a smaller delivery inner diameter in the collapsed delivery configuration.

The support frame can include a shape memory alloy with an Austenite finish temperature less than approximately 30 degrees Celsius.

The support frame can heat set to have the expanded inner diameter greater than an inner diameter of the collapsed delivery configuration.

The distal end of the support frame in the expanded deployed configuration can have a circular profile including a center radially offset from the longitudinal axis.

The distal end of the support frame in the collapsed delivery configuration can have a substantially circular cross section with a center substantially coincident with the longitudinal axis.

The support frame can include an axial length being less in the expanded delivery configuration than in the collapsed delivery configuration.

The support frame can include a maximum outer diameter in the expanded deployed configuration less than an inner diameter of a target vessel at a treatment site.

The distally unconnected peak of each of the hoop ribs can move proximally when the support frame is in compression during clot ingestion.

At least a portion of each of the hoop ribs can reside in a plane forming an acute angle with respect to the longitudinal axis.

The support frame can include one or more connector ribs extending from a ring member connected to a proximal end of the support frame, the connector ribs diverging from the ring member in an offset plane substantially perpendicular to the offset plane of the hoop ribs.

At least one of the series of hoop ribs can be connected proximally to the connector ribs, and at least one of the series of hoop ribs can be connected proximally to the ring member.

The support frame can include one or more support ribs extending from the ring member in a plane substantially parallel to the offset plane of the connector ribs. The support ribs can be free from a connection point with any of the series of hoop ribs or the connector ribs.

The ring member can be configured to press fit over a braided section of a tubular catheter shaft. The support frame can include a proximal collar at a proximal end. The proximal collar can include the ring member circumferentially divided by at least one seam.

Other aspects of the present disclosure will become apparent upon reviewing the following detailed description in conjunction with the accompanying figures. Additional features or manufacturing and use steps can be included as would be appreciated and understood by persons skilled in the pertinent art.

### BRIEF DESCRIPTION OF THE DRAWINGS

The above and further aspects of this disclosure are further discussed with reference to the following description in conjunction with the accompanying drawings, in which like numerals indicate like structural elements and features in various figures. The drawings are not necessarily to scale, emphasis instead being placed upon illustrating principles of the invention defined in the claims. The figures depict one or more implementations of the inventive devices, by way of example only, not by way of limitation. It is expected that those of skill in the pertinent art can conceive of and combine elements from multiple figures to better suit the needs of the user.
FIG. 1A is a perspective view of a clot retrieval catheter expandable support frame in a collapsed delivery configuration, according to aspects of the present invention.
FIG. 1B is a side view of the clot retrieval catheter expandable support frame of FIG. 1A, according to aspects of the present invention.
FIG. 1C is a top view of the clot retrieval catheter expandable support frame of FIGS. 1A and 1B, according to aspects of the present invention.
FIG. 1D is a cross-sectional view of the clot retrieval catheter expandable support frame of FIGS. 1A-1C, according to aspects of the present invention.
FIG. 1E is a cross-sectional view of the clot retrieval catheter expandable support frame of FIGS. 1A-1D, according to aspects of the present invention.
FIG. 2A is a perspective view of a clot retrieval catheter expandable support frame in a collapsed delivery configuration, according to aspects of the present invention.
FIG. 2B is a top view of the clot retrieval catheter expandable support frame of FIG. 2A, according to aspects of the present invention.
FIG. 2C is a side view of the clot retrieval catheter expandable support frame of FIGS. 2A and 2B, according to aspects of the present invention.
FIG. 2D is a side view of a clot retrieval catheter expandable support frame in a collapsed delivery configuration, according to aspects of the present invention.
FIG. 3A is a perspective view of a clot retrieval catheter expandable support frame in a collapsed delivery configuration, according to aspects of the present invention.
FIG. 3B is a top view of the clot retrieval catheter expandable support frame of FIG. 3A, according to aspects of the present invention.
FIG. 3C is a side view of the clot retrieval catheter expandable support frame of FIGS. 3A and 3B, according to aspects of the present invention.
FIG. 3D is a side view of a clot retrieval catheter expandable support frame in a collapsed delivery configuration, according to aspects of the present invention.
FIG. 3E is a side view of a clot retrieval catheter expandable support frame in a collapsed delivery configuration, according to aspects of the present invention.
FIG. 3F is a side view of a clot retrieval catheter expandable support frame in a collapsed delivery configuration, according to aspects of the present invention.
FIG. 3G is a side view of a clot retrieval catheter expandable support frame in a collapsed delivery configuration, according to aspects of the present invention.
FIG. 4A is a perspective view of a clot retrieval catheter expandable support frame in a collapsed delivery configuration, according to aspects of the present invention.
FIG. 4B is a top view of the clot retrieval catheter expandable support frame of FIG. 4A, according to aspects of the present invention.
FIG. 4C is a side view of the clot retrieval catheter expandable support frame of FIGS. 4A and 4B, according to aspects of the present invention.
FIG. 5A is a perspective view of a clot retrieval catheter expandable support frame in a collapsed delivery configuration, according to aspects of the present invention.
FIG. 5B is a top view of the clot retrieval catheter expandable support frame of FIG. 5A, according to aspects of the present invention.
FIG. 5C is a side view of the clot retrieval catheter expandable support frame of FIGS. 5A and 5B, according to aspects of the present invention.
FIG. 6A is a perspective view of a clot retrieval catheter expandable support frame in a collapsed delivery configuration, according to aspects of the present invention.
FIG. 6B is a top view of the clot retrieval catheter expandable support frame of FIG. 6A, according to aspects of the present invention.
FIG. 6C is a side view of the clot retrieval catheter expandable support frame of FIGS. 6A and 6B, according to aspects of the present invention.
FIG. 7 is a side view of a clot retrieval catheter tip expandable support frame profile, according to aspects of the present invention.
FIG. 8 is a side view of a clot retrieval catheter tip expandable support frame profile, according to aspects of the present invention.
FIG. 9 is a diagram of a clot retrieval catheter tip with an expandable support frame being advanced through the vasculature, according to aspects of the present invention.
FIGS. 10A-10C are illustrations of example treatment steps that can be performed using a clot retrieval catheter tip expandable support frame, according to aspects of the present invention.
FIGS. 11A-11C are illustrations of example treatment steps that can be performed using a clot retrieval catheter tip expandable support frame, according to aspects of the present invention.

### DETAILED DESCRIPTION

Specific examples of the present invention are now described in detail with reference to the Figures, where identical reference numbers indicate elements which are functionally similar or identical. The examples address many of the deficiencies associated with traditional clot retrieval aspiration catheters, such as poor or inaccurate deployment to a target site and ineffective clot removal.

The designs herein, illustrating various configurations of catheter tip support frames, can be incorporated into an aspiration clot retrieval catheter with a membrane cover or jacket encapsulation, proximal shaft, large bore lumen, and a distal low shear tip (LST) that can expand to a diameter larger than the nominal diameter when it interacts with an ingested clot or stentriever. The designs herein can also be pre-expanded and heat set to incorporate into a collapsible super bore (CSB) catheter with a membrane cover and proximal shaft to provide a catheter that collapses for delivery through a guide catheter and expands when exiting the guide catheter to be advanced to a target vessel for aspiration of a clot.

The designs herein can have a proximal elongate body for the shaft of the catheter, and a distal tip with an expanding inner frame to give the tip atraumatic properties. That is, the expanding inner frame is capable of easily and repeatedly collapsing for delivery and expanding locally either under loading from the clot (when used in conjunction with an LST catheter), or by being heat set (when used in conjunction with a CSB catheter), thereby enabling the catheter tip to expand beyond the nominal diameter to ingest a clot. The expanding inner frame can have a proximal ring for attaching to a braided catheter shaft, and can have an offset mouth allowing for a larger opening for clot retrieval and reduced stiffness for easier expansion. This management of the clot during ingestion can significantly reduce shearing of the clot. The catheter's design can be sufficiently flexible to navigate highly tortuous areas of the anatomy and be able to recover its shape to maintain the inner diameter of the lumen when displaced in a vessel.

This innovation of utilizing the clot itself to expand the tip section as needed allows for much improved clot handling and less shearing over traditional designs. The nominal, non-expanded outer diameter maximizes distal access reach like a standard fixed-mouth catheter. Once a clot is subsequently ingested, accommodating stiff, fibrin-rich portions of the clot through additional radial expansion can gradually compress the clot such that there is significantly less clot shearing than catheters that lack this capability. Further, the conformable nature of the tip allows it to be advanced atraumatically past calcified lesions without dislodging plaque material.

Accessing the various vessels within the vascular system, whether they are coronary, pulmonary, or cerebral vessels, involves well-known procedural steps and the use of a number of conventional, commercially-available accessory products. These products, such as angiographic materials, mechanical thrombectomy devices, microcatheters, and guidewires are widely used in laboratory and medical procedures. When these products are employed in conjunction with the devices of this invention in the description below, their function and exact constitution are not described in detail. Additionally, while the description is in many cases in the context of thrombectomy treatments in intercranial arteries, the disclosure may be adapted for other procedures and in other body passageways as well.

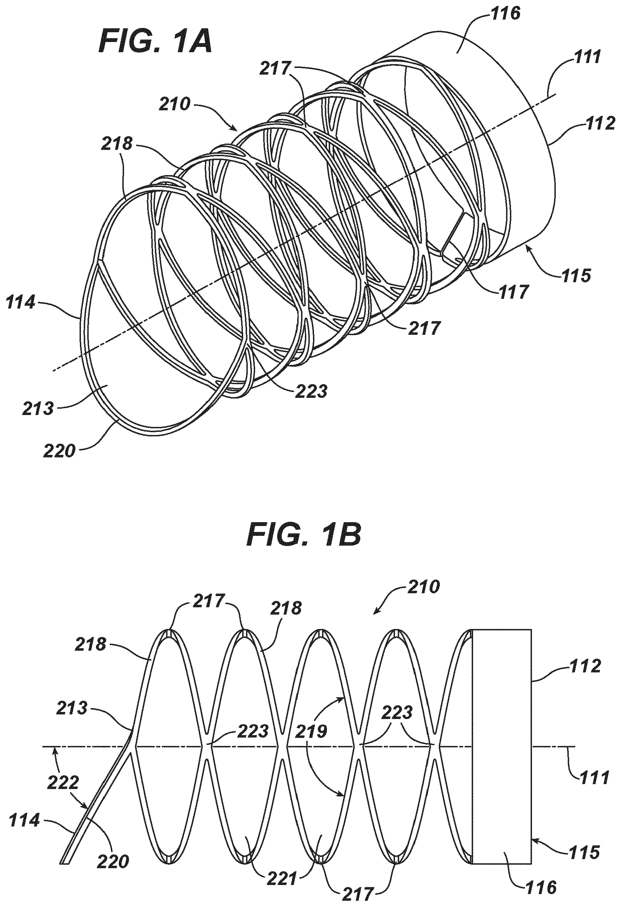

Turning to the figures, FIGS. 1A-1E illustrate an example expandable support frame 210 for use in a clot retrieval catheter tip. When used in conjunction with an LST catheter, support frame 210 can be manufactured using super elastic nickel titanium or Nitinol (NiTi), shape memory NiTi, or stainless steel. While shape memory material is not required for an LST catheter, it can provide an added benefit of enabling the support frame 210 to recover its shape if distorted during use, or if support frame 210 is expanded by a clot during a first retrieval pass and needs to be easily recovered to track back through a guide sheath for making a second retrieval pass. When used in conjunction with a CSB catheter, shape memory material enables support frame 210 to self-expand when it exits the distal end of the catheter tip and approaches the clot for retrieval. The shape memory alloy used can include an Austenite finish temperature less than approximately 30 degrees Celsius.

Support frame 210 can be manufactured by taking raw tubing, of a material as discussed above, and laser cutting the material to produce the desired configuration of support frame 210, as will be described further below. The raw tubing can have an outer diameter of approximately 2.00 millimeters, a wall thickness of approximately 0.05 millimeters, and an inner diameter of approximately 1.90 millimeters. Support frame 210 can also be manufactured to have light electropolishing or other such finish. A matte finish can provide a benefit of enhancing adhesion to a polymer catheter jacket.

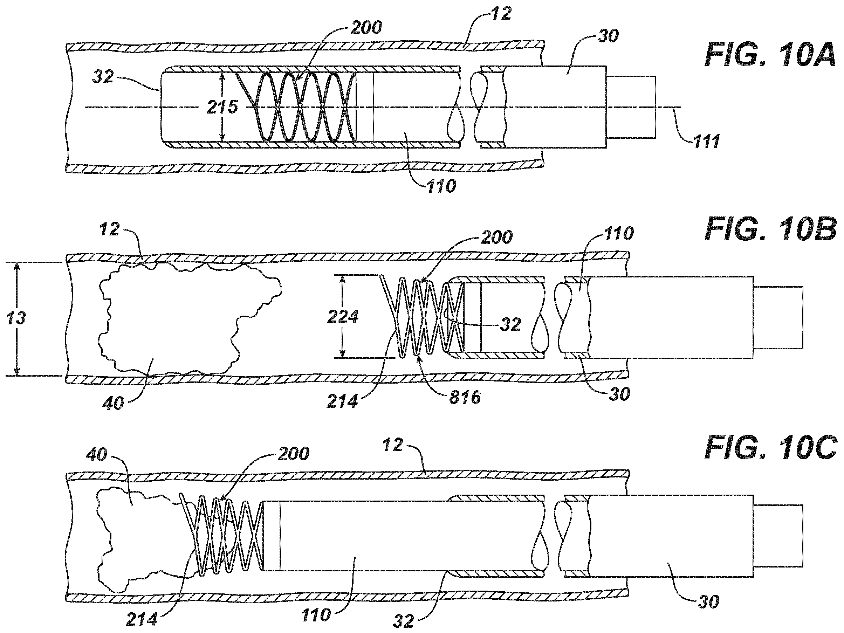

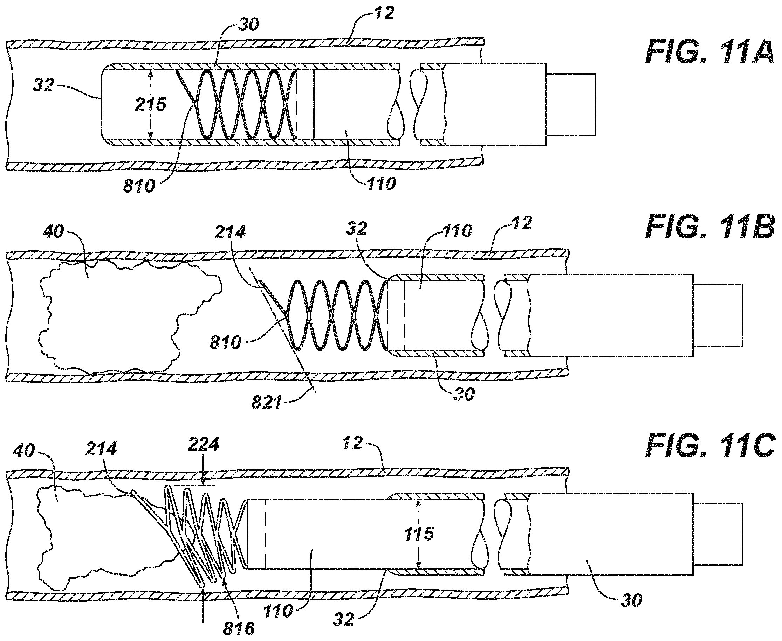

The support frame 210 can include a longitudinal axis 111, a collapsed delivery configuration (FIGS. 1A-1C), an expanded deployed configuration (FIGS. 10B-10C or 11C), and a plurality of interconnected struts 218 defining an axial series of expansion cells 221. The interconnected struts 218 can be joined at opposing pairs of x-connectors 217 that are spaced apart by 180 degrees about the longitudinal axis 111. Each opposing pair of x-connectors 217 can be rotated 90 degrees about the longitudinal axis 111 with respect to an adjacent opposing pair of x-connectors 217, as particularly shown in FIG. 1A. Such rotational configuration can provide enhanced flexibility of support frame 210 across multiple planes, compared to many conventional support frame designs.

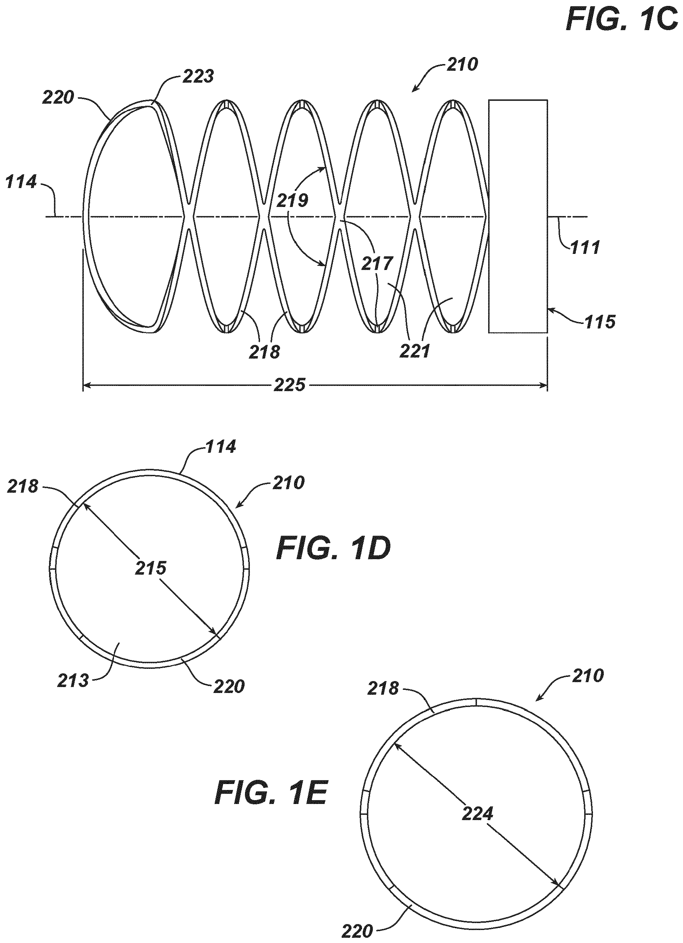

As particularly shown in FIGS. 1B-1C, support frame 210 can include a distal strut angle 219 between struts 218 on opposing sides of longitudinal axis 111. Distal strut angle 219 can be configured as an obtuse angle such that it can provide enhanced flexibility and compressibility during clot retrieval, as described further below. The struts 218 can also include a curvilinear profile to aid in flexibility of the support frame 210. That is, the series of expansion cells 221 can shorten longitudinally when the support frame 210 is placed in compression (FIGS. 10C and 11C). As such, support frame 210 can include an axial length 225 (FIG. 1C) that can be less in the expanded deployed configuration than in the collapsed delivery configuration, as discussed further below with respect to FIGS. 10A-10C and 11A-11C.

As particularly shown in FIG. 1B, the axial series of expansion cells 221 can include opposing pairs of cells spaced 180 degrees apart about the longitudinal axis 111. Each opposing pair of cells can also be rotated 90 degrees about the longitudinal axis 111 with respect to an adjacent pair of opposing cells.

When in the collapsed delivery configuration, a distal end 114 of the support frame 210 can include a substantially circular cross section with a center substantially coincident with the longitudinal axis, as particularly shown in FIGS. 1A-1B, and as further described below.

The support frame 210 can also include a proximal collar 115 at its proximal end 112. The proximal collar 115 can be used for attaching the support frame 210 to a braided shaft of a clot retrieval catheter. The proximal collar 115 can include a ring member 116 that is circumferentially divided by at least one seam 117 (FIG. 1A), such as an angled seam, which aids in assembly of the proximal collar 115. In some embodiments, ring member 116 may be configured to connect to a braided section of a tubular catheter shaft. For example, ring member 116 may be configured to press fit over the braided section, or may be laser welded to the braided section (e.g., fitting under or over the braided section). In other examples, ring member 116 may be adhesively bonded to the braided section. In some embodiments, rather than a braided section, ring member 116 may be formed integrally with and/or laser welded to a laser-cut proximal catheter support structure.

As shown in FIGS. 1A-1B, support frame 210 can include a mouth 213 at a distal end 114, the mouth 213 having an offset mouth strut 220. At least a portion of the offset mouth strut 220 can reside in a plane forming an acute angle 222 with respect to the longitudinal axis 111. As shown, offset mouth strut 220 can be connected to one or more struts 218 by one or more y-connectors 223. A benefit of the offset mouth strut 220 is that it can allow for a larger space for the clot to enter into the catheter tip. This can provide easier and more efficient clot retrieval from the treatment site and can reduce the risk of clot shearing or breakage during retrieval, compared to many conventional support frame designs. The offset mouth may also allow for an increased clot grip force due to the larger area of the mouth compared to a circular cross section of the catheter.

As shown in FIGS. 1D-1E, the support frame 210 can include a collapsed inner diameter 215 (FIG. 1D) when in the collapsed delivery configuration, and a larger inner diameter 224 (FIG. 1E) in the expanded deployed configuration when the support frame 210 is placed in compression. The collapsed inner diameter 215 enables support frame 210 to fit within guide sheath 30 (FIG. 10A) when the catheter is being navigated through a vessel 12 toward a treatment site. As will be described further below with respect to FIGS. 11A-11C, when used in conjunction with an LST catheter, support frame 210 can maintain its collapsed inner diameter 215 as it exits the distal end 32 of the guide sheath 30, and then expand from the collapsed inner diameter 215 to the expanded inner diameter 224 when impinged by an ingested clot 40. Alternatively, when used in conjunction with a CSB catheter, as will be described further below with respect to FIGS. 10A-10C, support frame 210 can be heat set such that it has expanded inner diameter 224, greater than the collapsed inner diameter 215, as soon as support frame 210 exits the distal end 32 of the guide sheath 30. In some embodiments, when used in conjunction with either an LST or CSB catheter, for example, when support frame 210 is in its expanded deployed configuration (FIGS. 10B-10C and 11C), its maximum outer diameter can be less than an inner diameter 13 of the target vessel 12 at a treatment site, such that support frame 210 can advance distally toward clot 40 independently from and without sealing to the vessel 12. In some embodiments, when used in conjunction with a CSB catheter, for example, the maximum outer diameter of support frame 210 may be greater than the inner diameter 13 of the target vessel 12. In such embodiments, support frame 210 may be configured to self-collapse when advanced distally through smaller vessels such that it can provide a seal within the vessels.

With respect to the various support frames disclosed herein, the inner and outer diameters of the support frames when in a collapsed delivery configuration versus an expanded deployed configuration may depend on the size of the catheter being used in conjunction with the support frame.

When using a support frame in conjunction with a 5Fr low shear tip, for example, a collapsed inner diameter may be approximately 1.3716 mm (0.054 inches), while a collapsed outer diameter may be approximately 1.6764 mm (0.066 inches). This size support frame, in its collapsed configuration, may be used in target vessels having an approximately 1.7 millimeter inner diameter, while in its expanded configuration, may expand to seal in target vessels having an approximately 2.2 millimeter inner diameter.

When using a support frame in conjunction with a 6Fr low shear tip, for example, a collapsed inner diameter may range from approximately 1.7272 mm (0.068 inches) to approximately 1.8796 mm (0.074 inches), while a collapsed outer diameter may range from approximately 2.032 mm (0.080 inches) to approximately 2.1844 mm (0.086 inches). This size support frame, in its collapsed configuration, may be used in target vessels having an approximately 2.0 to 2.2 millimeter inner diameter, while in its expanded configuration, may expand to seal in target vessels having an approximately 3.5 millimeter inner diameter.

When using a support frame in conjunction with an 8Fr low shear tip, for example, a collapsed inner diameter may range from approximately 2.0828 mm (0.082 inches) to approximately 2.413 mm (0.095 inches), while a collapsed outer diameter may range from approximately 2.3876 mm (0.094 inches) to approximately 2.921 mm (0.115 inches). This size support frame may be used in target vessels having an approximately 2.4 to 2.9 millimeter inner diameter, while in its expanded configuration, may expand to seal in target vessels having an approximately 5.0 millimeter inner diameter.

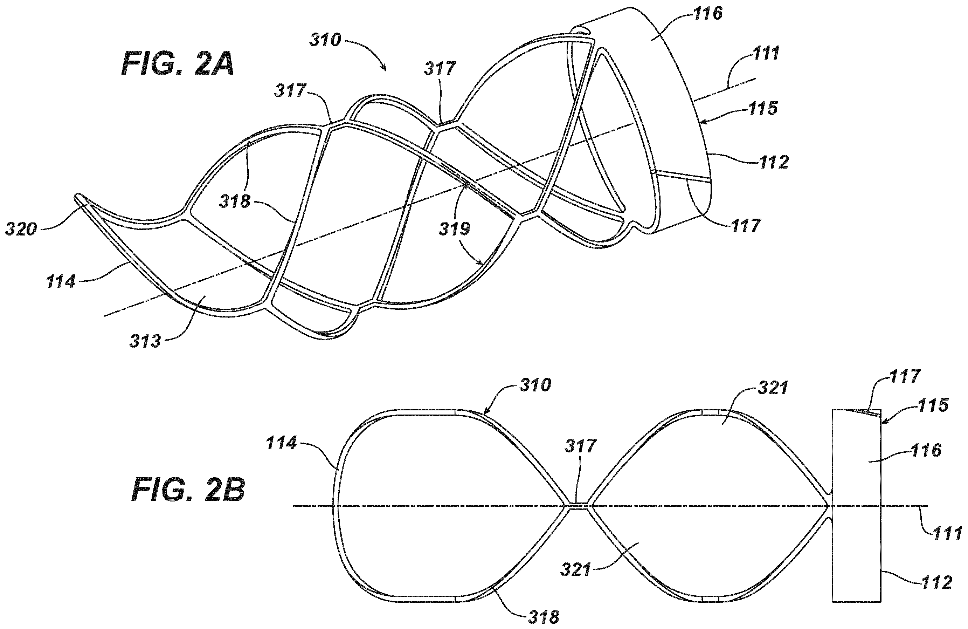

FIGS. 2A-2D illustrate another example expandable support frame 310 for use in a clot retrieval catheter tip. Support frame 310 can include one or more features that are the same as or similar to those described above with respect to support frame 210. Further, the manufacturing of support frame 310 can be the same as or similar to that of support frame 210, as discussed above. Support frame 310 can also be manufactured to be electropolished, pickled to roughen the surface of support frame 310, and/or heat treated to have an oxide layer finish.

Support frame 310 can include longitudinal axis 111, a collapsed delivery configuration (FIGS. 2A-2C), an expanded deployed configuration, and a plurality of interconnected struts 318 defining an axial series of expansion cells 321. The interconnected struts 318 can be joined at opposing pairs of x-connectors 317 that are spaced apart by 180 degrees about the longitudinal axis 111. Each opposing pair of x-connectors 317 can be rotated 90 degrees about the longitudinal axis 111 with respect to an adjacent opposing pair of x-connectors 317. Such rotational configuration can provide enhanced flexibility of support frame 310 across multiple planes, compared to many conventional support frame designs.

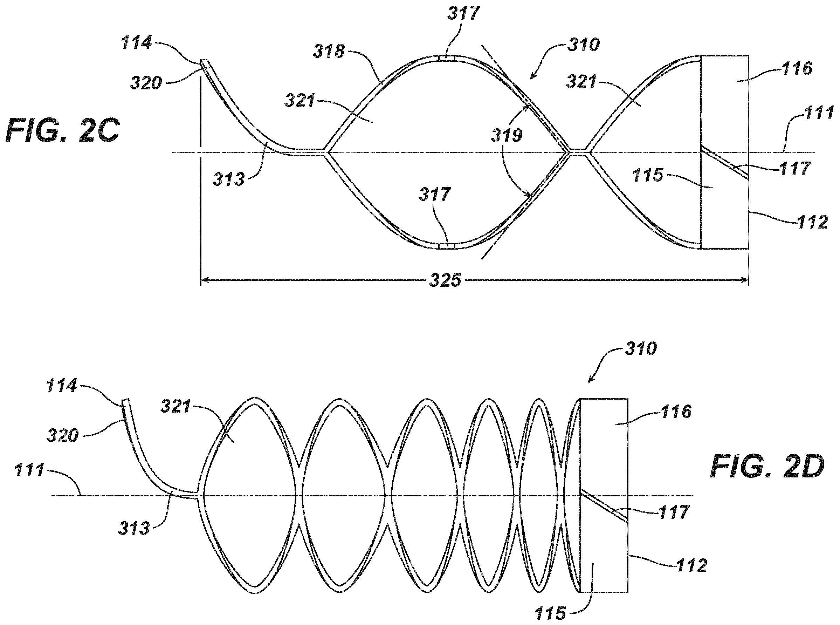

Support frame 310 can also include a distal strut angle 319 between struts 318 on opposing sides of longitudinal axis 111, as particularly shown in FIG. 2C. Distal strut angle 319 can be configured as an acute angle due to its increased length of x-connector struts 317. An acute distal strut angle 319 can provide increased expansion of support frame 310, and the increased length of x-connector struts 317 can provide for less restricted bending, compared to many conventional support frame designs.

As particularly shown in FIG. 2C, the interconnected struts 318 can also include a curvilinear profile to aid in flexibility of the support frame 310. That is, the series of expansion cells 321 can shorten longitudinally when the support frame 310 is placed in compression. As such, as shown in FIG. 2C, support frame 310 can include an axial length 325 that can be less in the expanded deployed configuration than in the collapsed delivery configuration, as discussed further below with respect to support frame 210 in FIGS. 10A-10C and 11A-11C.

As also particularly shown in FIG. 2C, the axial series of expansion cells 321 can include opposing pairs of cells spaced 180 degrees apart about the longitudinal axis 111. Each opposing pair of cells can also be rotated 90 degrees about the longitudinal axis 111 with respect to an adjacent pair of opposing cells. In some embodiments, the support frame 310 may be modified to have variable spacing with respect to the expansion cells 321, as particularly shown in FIG. 2D. Variable cell spacing may allow for gradual expansion during compression of the support frame 310, and may provide enhanced lateral flexibility at the proximal end of the support frame 310, e.g., near the proximal collar 115, as discussed further below. It should be appreciated that any of the various support frames disclosed herein may be modified to have variable cell spacing.

When in the collapsed delivery configuration, a distal end 114 of the support frame 310 can include a substantially circular cross section with a center substantially coincident with the longitudinal axis, as particularly shown in FIGS. 2A and 2C, and as further described below.

The support frame 310 can also include a proximal collar 115 at its proximal end 112. The proximal collar 115 can be used for attaching the support frame 310 to a braided shaft of a clot retrieval catheter. The proximal collar 115 can include a ring member 116 that is circumferentially divided by at least one seam 117, such as an angled seam, which aids in assembly of the proximal collar 115.

As shown in FIGS. 2A and 2C, support frame 310 can include a mouth 313 at a distal end 114, the mouth 313 having an offset mouth strut 320. At least a portion of the offset mouth strut 320 can reside in a plane forming an acute angle 322 with respect to the longitudinal axis 111. A benefit of the offset mouth strut 320 is that it can allow for a larger space for the clot to enter into the catheter tip. This can provide easier and more efficient clot retrieval from the treatment site and can reduce the risk of clot shearing or breakage during retrieval, compared to many conventional support frame designs.

Similar to support frame 210, as described above with respect to FIGS. 1D-1E, support frame 310 can include a collapsed inner diameter when in the collapsed delivery configuration, and a larger inner diameter in the expanded deployed configuration when the support frame 310 is placed in compression. In some embodiments, when wsed in conjunction with either an LST or CSB catheter, for example, when support frame 310 is in its expanded deployed configuration, its maximum outer diameter may be less than an inner diameter 13 of a target vessel 12 at a treatment site, such that support frame 310 can advance distally toward clot 40 independently from and without sealing to the vessel 12. In some embodiments, when used in conjunction with a CSB catheter, for example, support frame 310 may have an outer diameter greater than the inner diameter of the target vessel 12, and be configured to self-collapse when advancing distally through a target vessel. For example, the catheter tip, including support frame 310, may be configured such that the radial collapsing force is reduced when the support frame 310 is advanced through a vessel 12 that may reduce distally with respect to its inner diameter, while still keeping the crush resistance high enough that the tip remains open on full vacuum when blocked. This may allow a catheter with support frame 310 to be used in a wide range of vessel sizes.

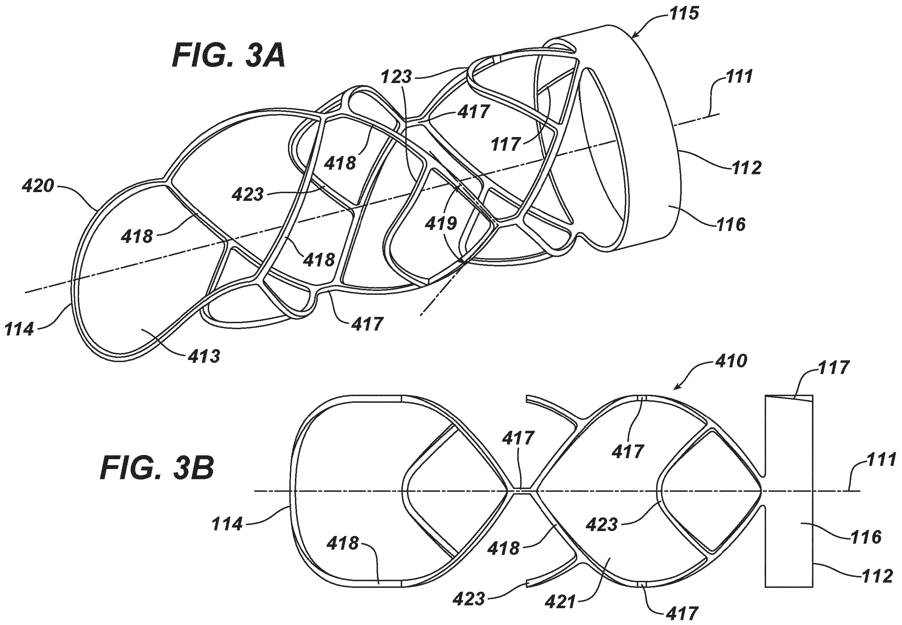

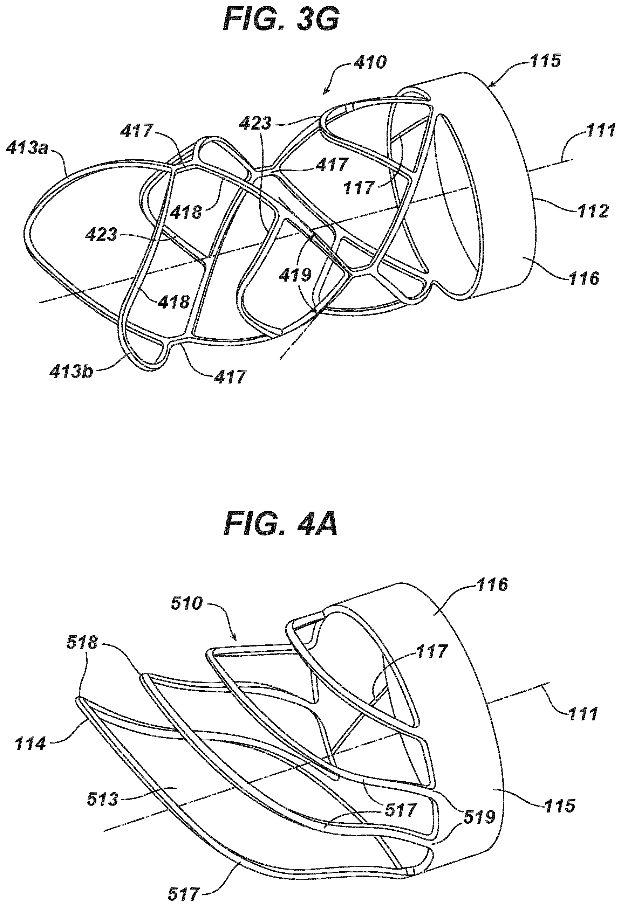

FIGS. 3A-3G illustrate another example expandable support frame 410 for use in a clot retrieval catheter tip. Support frame 410 can include one or more features that are the same as or similar to those described with respect to support frames 210 and 310. Further, the manufacturing of support frame 410 can be the same as or similar to that of support frames 210 and 310. Support frame 410 can also be manufactured to be electropolished, pickled to roughen the surface of support frame 410, and/or heat treated to have an oxide layer finish.

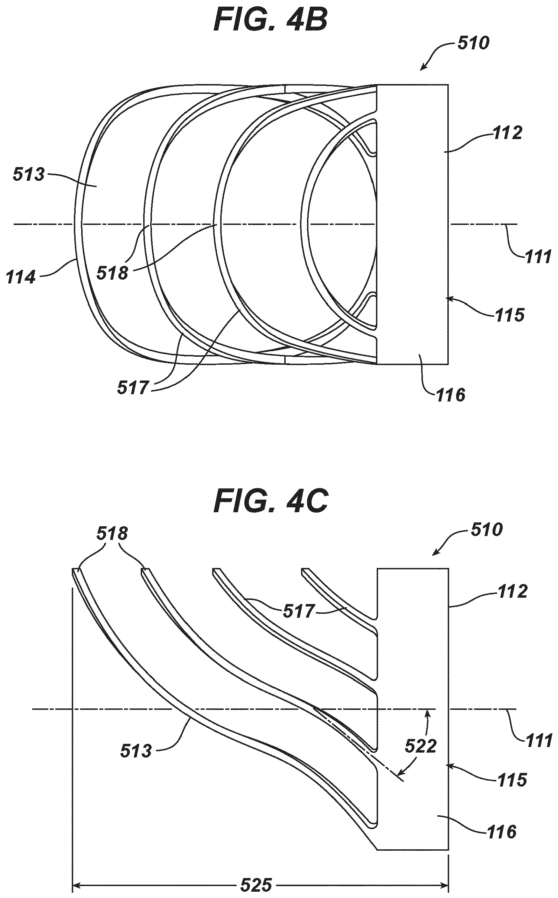

Support frame 410 can include longitudinal axis 111, a collapsed delivery configuration (FIGS. 4A-4C), an expanded deployed configuration, and a plurality of interconnected struts 418 defining an axial series of expansion cells 421. The interconnected struts 418 can be joined at opposing pairs of x-connectors 417 that are spaced apart by 180 degrees about the longitudinal axis 111. Each opposing pair of x-connectors 417 can be rotated 90 degrees about the longitudinal axis 111 with respect to an adjacent opposing pair of x-connectors 417. Such rotational configuration also can provide enhanced flexibility of support frame 410 across multiple planes, compared to many conventional support frame designs.

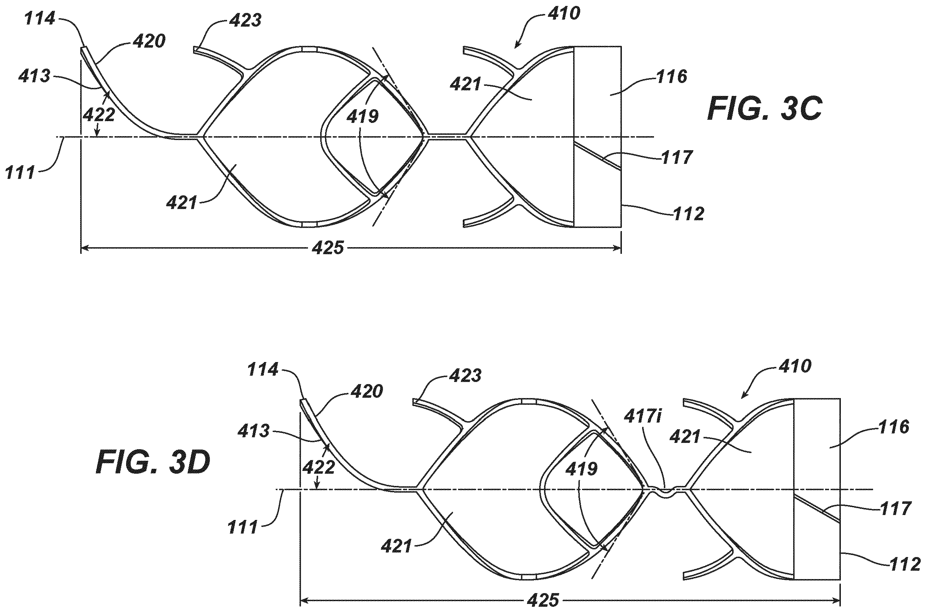

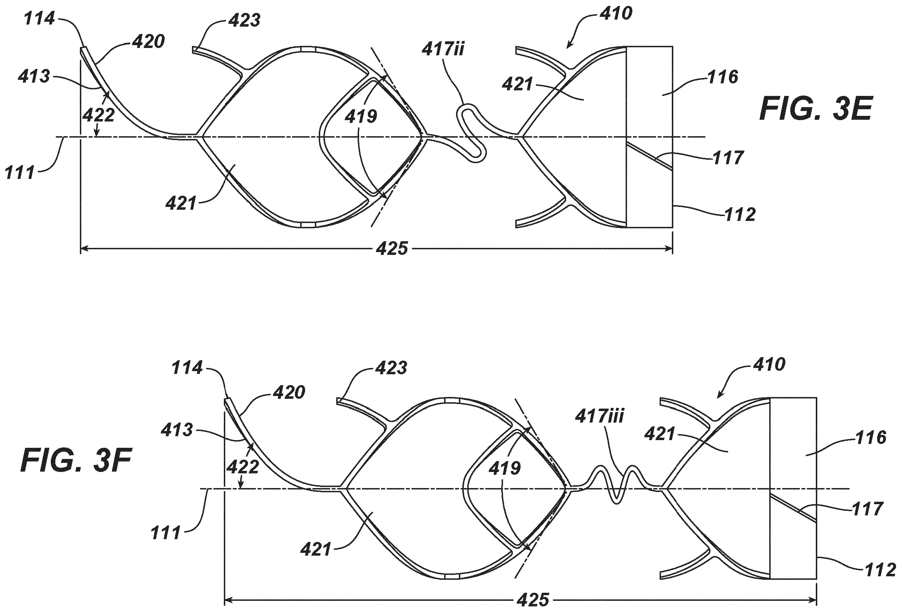

In some embodiments, interconnected struts 418 may be joined at opposing pairs of u-connectors 417i (as shown in FIG. 3D), s-connectors 417ii (as shown in FIG. 3E), and/or m/w connectors 417iii (as shown in FIG. 3F). The use of different connector types and/or shapes between interconnected struts 418 may provide for variations in lateral flexibility of the support frame 410. It should be appreciated that different connector types and/or shapes may be used in the various support frames disclosed herein.

Support frame 410 can also include a distal strut angle 419 between struts 418 on opposing sides of longitudinal axis 111, as particularly shown in FIGS. 3C-3F. Distal strut angle 419 can be configured as an acute angle due to its increased length of x-connector struts 417 (FIG. 3A). The acute distal strut angle 419 can provide for increased expansion of support frame 410, and the increased length of x-connector struts 417 can provide for less restricted bending, compared to many conventional support frame designs.

Support frame 410 can also include one or more additional v-struts 423, as shown in FIGS. 3A-3F. The additional v-struts 423 can allow for increased radial force and crush resistance, especially in light of the acute distal strut angle 419, compared to many conventional support frame designs.

As particularly shown in FIG. 3B, the interconnected struts 418 can also include a curvilinear profile to aid in flexibility of the support frame 410. That is, the series of expansion cells 421 can shorten longitudinally when the support frame 410 is placed in compression. As such, as shown in FIGS. 3C-3F, support frame 410 can include an axial length 425 which can be less in the expanded deployed configuration than in the collapsed delivery configuration, as discussed further below with respect to support frame 210 in FIGS. 10A-10C and 11A-11C.

As also particularly shown in FIG. 3C-3F, the axial series of expansion cells 421 can include opposing pairs of cells spaced 180 degrees apart about the longitudinal axis 111. Each opposing pair of cells can also be rotated 90 degrees about the longitudinal axis 111 with respect to an adjacent pair of opposing cells.

When in the collapsed delivery configuration, a distal end 114 of the support frame 410 can include a substantially circular cross section with a center substantially coincident with the longitudinal axis, as particularly shown in FIGS. 3A and 3C-3F, and as further discussed below.

The support frame 410 can include a proximal collar 115 at its proximal end 112. The proximal collar 115 can be used for attaching the support frame 410 to a braided shaft of a clot retrieval catheter. The proximal collar 115 can include a ring member 116 that is circumferentially divided by at least one seam 117, such as an angled seam, which aids in assembly of the proximal collar 115.

As shown in FIGS. 3A and 3C-3F, support frame 410 can include a mouth 413 at a distal end 114, the mouth 413 having an offset mouth strut 420. At least a portion of the offset mouth strut 420 can reside in a plane forming an acute angle 422 with respect to the longitudinal axis 111. A benefit of the offset mouth strut 420 is that it can allow for a larger space for the clot to enter into the catheter tip. This can provide easier and more efficient clot retrieval from the treatment site and can reduce the risk of clot shearing or breakage during retrieval, compared to many conventional support frame designs. For firm fibrin rich clots that may not be aspirated entirely through the inner diameter of the catheter, the offset mouth may provide a larger surface area to increase the clot grip force and enable the tip to hold onto the clot and retract it from the vessel and into a larger guide catheter positioned more proximally in the vasculature.

In some embodiments, mouth 413 of support frame 410 (or the mouth of any other support frame disclosed herein) may be provided in two planes, such as in the shape of two opposing struts, 413a and 413b. The two opposing struts 413a, 413b may be configured to follow the shape of the distal-most struts of the support frame 410 such that the surface area of the mouth 413 may be provided in two planes. In such embodiments, the mouth 413 may have an increased surface area over a shorter length, and may allow for aspiration from two opposing sides of the distal tip of the catheter.

Similar to support frames 210 and 310, support frame 410 can include a collapsed inner diameter when in the collapsed delivery configuration, and a larger inner diameter in the expanded deployed configuration when the support frame 410 is placed in compression. In some embodiments, when used in conjunction with either an LST or CSB catheter, for example, when support frame 410 is in its expanded deployed configuration, its maximum outer diameter may be less than an inner diameter 13 of a target vessel 12 at a treatment site, such that support frame 410 can advance distally toward clot 40 independently from and without sealing to the vessel 12. In some embodiments, when used in conjunction with a CSB catheter, for example, support frame 410 may have an outer diameter greater than the inner diameter of the target vessel 12, and be configured to self-collapse when advancing distally through a target vessel. For example, the catheter tip, including support frame 410, may be configured such that the radial collapsing force is reduced when the support frame 410 is advanced through a vessel 12 that may reduce distally with respect to its inner diameter, while still keeping the crush resistance high enough that the tip remains open on full vacuum when blocked. This may allow a catheter with support frame 410 to be used in a wide range of vessel sizes.

FIGS. 4A-4C illustrate another example expandable support frame 510 for use in a clot retrieval catheter tip. Support frame 510 can include one or more features that are the same as or similar to those described about with respect to support frames 210, 310, and 410. The manufacturing of support frame 510 when used in conjunction with either an LST or CSB catheter can be the same as or similar to that of support frames 210, 310, and 410, as discussed above. Support frame 510 can also be manufactured to be electropolished, pickled to roughen the surface of support frame 510, and/or heat treated to have an oxide layer finish.

Support frame 510 can include longitudinal axis 111, a collapsed delivery configuration (FIGS. 4A-4C), an expanded deployed configuration, and an axial series of hoop ribs 517 extending in planes offset from the longitudinal axis 111. The hoop ribs 517 can include a curvilinear profile, a non-planar cross section, and a distally unconnected peak 518. Support frame 510 can also include a mouth 513 having a larger expanded inner diameter when support frame 510 is placed in compression (FIGS. 10C and 11C).

The distally unconnected peak 518 of each of the hoop ribs 517 can move proximally when the support frame 510 is in compression during clot ingestion. This feature can allow a clot 40 to more easily become lodged in support frame 510 during retrieval compared to many conventional support frame designs.

As particularly shown in FIG. 4C, at least a portion of each of the hoop ribs 517 can reside in a plane forming an acute angle 522 with respect to the longitudinal axis 111.

As shown in FIG. 4A, each of the hoop ribs 517 can also be connected to a proximal collar 115 at a connection point 519. This feature can aid in minimizing stiffness of support frame 510. The proximal collar 115 can be used for attaching support frame 510 to a braided shaft of a clot retrieval catheter. The proximal collar 115 can include a ring member 116 circumferentially divided by at least one seam 117, such as an angled seam, which aids in assembly of the proximal collar 115.

As shown in FIG. 4C, support frame 510 can include an axial length 525 which can be less in the expanded deployed configuration than in the collapsed delivery configuration, as discussed further below with respect to support frame 210 in FIGS. 10A-10C and 11A-11C.

Similar to support frame 210 as discussed above with respect to FIGS. 1D-1E, support frame 510 can include a larger expanded inner diameter 224 (FIG. 1E) when impinged radially by an ingested clot 40 in the expanded deployed configuration, and a smaller delivery inner diameter 215 (FIG. 1D) when in the collapsed delivery configuration. The collapsed inner diameter 215 enables support frame 210 to fit within guide sheath 30 (FIG. 10A) when the catheter is being navigated through a vessel 12 toward a treatment site. As will be described further below with respect to FIGS. 11A-11C, when used in conjunction with an LST catheter, support frame 510 can maintain its collapsed inner diameter 215 as it exits the distal end 32 of the guide sheath 30, and then expand from the collapsed inner diameter 215 to the expanded inner diameter 224 when impinged by an ingested clot 40. Alternatively, when used in conjunction with a CSB catheter, as will be described further below with respect to FIGS. 10A-10C, support frame 510 can be heat set such that it has expanded inner diameter 224, greater than the collapsed inner diameter 215, as soon as support frame 510 exits the distal end 32 of the guide sheath 30. Used in conjunction with either an LST or CSB catheter, when support frame 510 is in its expanded deployed configuration, its maximum outer diameter can be less than an inner diameter 13 of the target vessel 12 at a treatment site, such that support frame 510 can advance distally toward clot 40 independently from and without sealing to the vessel 12.

When in the collapsed delivery configuration, a distal end 114 of the support frame 510 can include a substantially circular cross section with a center substantially coincident with the longitudinal axis 111, as particularly shown in FIGS. 4A and 4C, and as further described below.

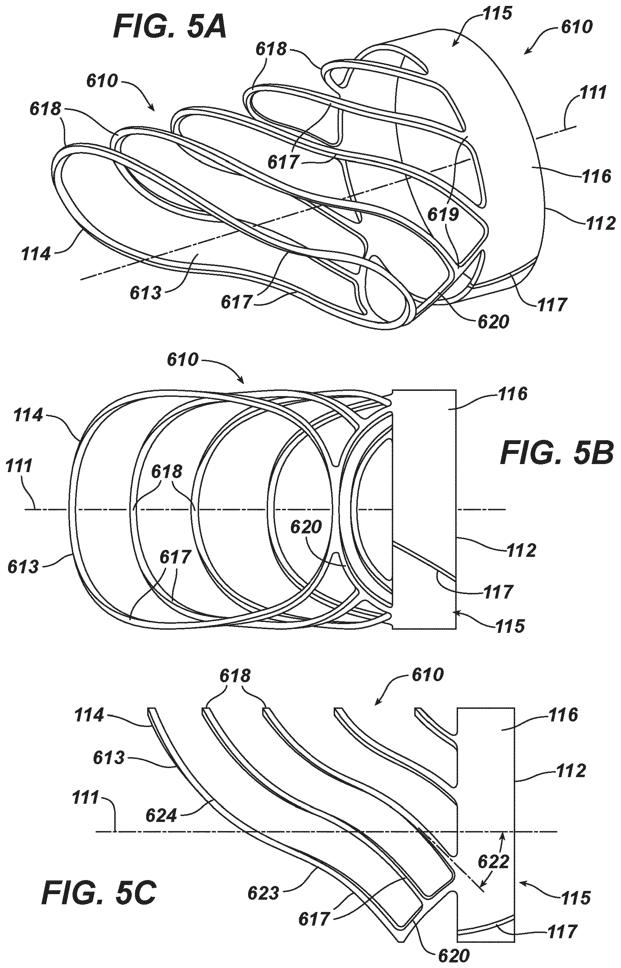

FIGS. 5A-5C illustrate another example expandable support frame 610 for use in a clot retrieval catheter tip. Support frame 610 can include one or more features that are the same as or similar to those described about with respect to support frames 210, 310, 410, and 510. The manufacturing of support frame 610 when used in conjunction with either an LST or CSB catheter can be the same as or similar to that of support frames 210, 310, 410, and 510, as discussed above. Support frame 610 can also be manufactured to be electropolished, pickled to roughen the surface of support frame 610, and/or heat treated to have an oxide layer finish.

Support frame 610 can include longitudinal axis 111, a collapsed delivery configuration (FIGS. 5A-5C), an expanded deployed configuration, and an axial series of hoop ribs 617 extending in planes offset from the longitudinal axis 111. The hoop ribs 617 can include a curvilinear profile, a non-planar cross section, and a distally unconnected peak 618. Support frame 610 can also include a mouth 613 having a larger expanded inner diameter when support frame 610 is placed in compression (FIGS. 10C and 11C).

The distally unconnected peak 618 of each of the hoop ribs 617 can move proximally when the support frame 610 is in compression during clot ingestion. This feature can allow a clot 40 to more easily become lodged in support frame 610 during retrieval compared to many conventional support frame designs.

As particularly shown in FIG. 5C, at least a portion of each of the hoop ribs 617 can reside in a plane forming an acute angle 622 with respect to the longitudinal axis 111.

As particularly shown in FIG. 5A, at least one hoop rib 617 can be directly connected to a proximal collar 115 at a connection point 619. One or more other hoop ribs 617 can instead be connected to one or more connector ribs 620 at one or more connection points 619. Connector ribs 620 can extend from a ring member 116 connected to a proximal end 112 of support frame 610. Connector ribs 620 can diverge from ring member 116 in an offset plane substantially perpendicular to the offset plane of the hoop ribs 617, as particularly shown in FIG. 5C. Connector ribs 620 can aid in increasing flexibility of support frame 610 during tacking and can allow for more symmetric expansion during clot retrieval compared to many conventional support frame designs.

As particularly shown in FIG. 5C, one or more of the hoop ribs 617 can also include a first curve 623 and a second curve 624 to aid in flexibility of support frame 610 during expansion and clot retrieval.

Similar to other support frames discussed herein, support frame 610 can also include an axial length which can be less in the expanded deployed configuration than in the collapsed delivery configuration, as discussed further below with respect to FIGS. 10A-10C and 11A-11C.

Similar to support frame 210 as discussed above with respect to FIGS. 1D-1E, support frame 610 can include a larger expanded inner diameter 224 (FIG. 1E) when impinged radially by an ingested clot 40 in the expanded deployed configuration, and a smaller delivery inner diameter 215 (FIG. 1D) of the collapsed delivery configuration. Support frame 610 can include one or more of the same or similar features as support frame 510 when being used in conjunction with an LST or CSB catheter, as discussed above.

When in the collapsed delivery configuration, a distal end 114 of the support frame 610 can include a substantially circular cross section with a center substantially coincident with the longitudinal axis 111, as particularly shown in FIGS. 5A and 5C, and as further described below.

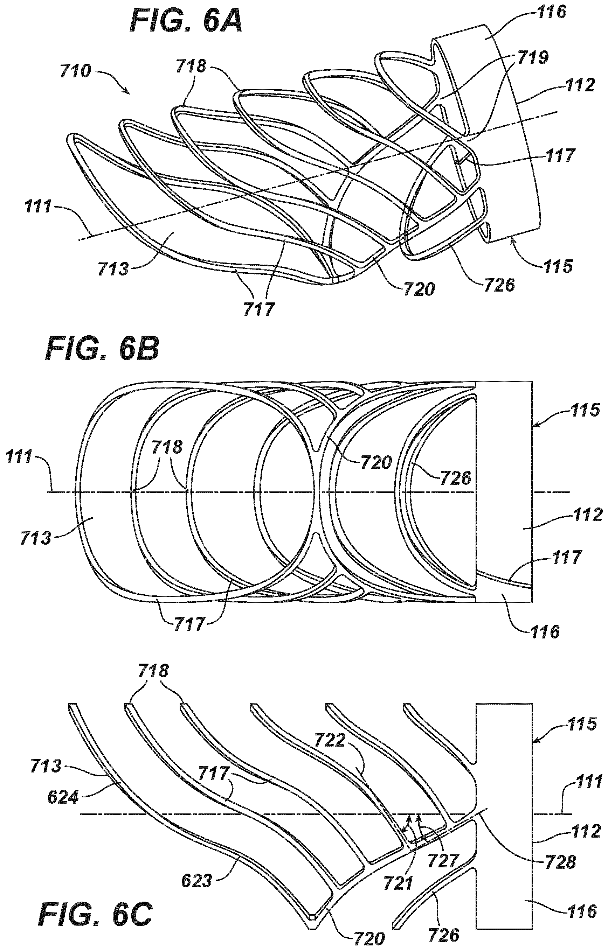

FIGS. 6A-6C illustrate another example expandable support frame 710 for use in a clot retrieval catheter tip. Support frame 710 can include one or more features that are the same as or similar to those described about with respect to support frames 210, 310, 410, 510, and 610. The manufacturing of support frame 710 when used in conjunction with either an LST or CSB catheter can be the same as or similar to that of support frames 210, 310, 410, 510, and 610 as discussed above. Support frame 710 can also be manufactured to be electropolished, pickled to roughen the surface of support frame 710, and/or heat treated to have an oxide layer finish.

Support frame 710 can include longitudinal axis 111, a collapsed delivery configuration (FIGS. 6A-6C), an expanded deployed configuration, and an axial series of hoop ribs 717 extending in planes offset from the longitudinal axis 111. The hoop ribs 717 can include a curvilinear profile, a non-planar cross section, and a distally unconnected peak 718. Support frame 710 can also include a mouth 713 having a larger expanded inner diameter when support frame 710 is placed in compression (FIGS. 10C and 11C).

The distally unconnected peak 718 of each of the hoop ribs 717 can move proximally when the support frame 710 is in compression during clot ingestion. This feature can allow a clot 40 to more easily become lodged in support frame 710 during retrieval compared to many conventional support frame designs.

As particularly shown in FIG. 6C, at least a portion of each of the hoop ribs 717 can reside in an offset plane 721 forming an acute angle 722 with respect to the longitudinal axis 111.

As particularly shown in FIG. 6A, at least one hoop rib 717 can be directly connected to a proximal collar 115 at a connection point 719. One or more other hoop ribs 717 can instead be connected to one or more connector ribs 720 at one or more connection points 719. Connector ribs 720 can extend from a ring member 116 connected to a proximal end 112 of support frame 710. Connector ribs 720 can diverge from ring member 116 in an offset plane 727 substantially perpendicular to the offset plane 721 of the hoop ribs 717, as particularly shown in FIG. 6C. Connector ribs 720 can aid in increasing flexibility of support frame 710 during tacking and can allow for more symmetric expansion during clot retrieval compared to many conventional support frame designs.

Support frame 710 can also include one or more support ribs 726 extending from the ring member 116 in a plane substantially parallel to the offset plane 727 of the connector ribs 720. The support ribs 726 can be free from a connection point with any of the series of hoop ribs 717 or the connector ribs 720, which can allow for a greater degree of expansion and more symmetric expansion compared to many conventional support frame designs.

As particularly shown in FIG. 6C, one or more of the hoop ribs 717 can also include a first curve 623 and a second curve 624 to aid in flexibility of support frame 710 during expansion and clot retrieval.

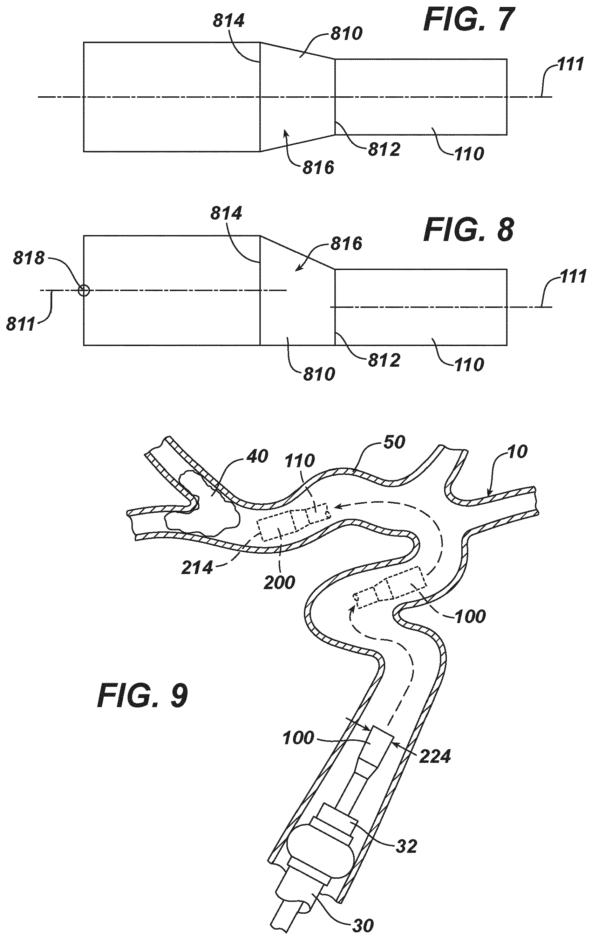

FIGS. 7 and 8 illustrate examples of a catheter shaft profile 110 when support frame(s) described herein are in their expanded deployed configurations and used in conjunction with either a CSB or an LST catheter.

FIG. 7 illustrates an example of a catheter shaft profile 110. The shaft profile 110 can include a circular profile symmetrical about the longitudinal axis 111. That is, the shaft 110 can include a tip section 810 including a proximal end 812, a distal end 814, and a funnel profile 816 disposed between the proximal end 812 and the distal end 814. The funnel profile 816, and sections distal and proximal to funnel profile 816, can be symmetrical about the longitudinal axis 111. The symmetrical nature of shaft profile 110 can be based on the support frame being heat set such that upon exiting the distal end 32 of the guide sheath 30 (FIG. 10B), the support frame can expand to an expanded inner diameter 224.

FIG. 8 illustrates another example of a catheter shaft profile 110. The funnel profile 816 can include a center 818 that is radially offset from the longitudinal axis 111. That is, center 818 can be centered with respect to offset axis 811, thereby configuring funnel profile 816 to be offset with respect to longitudinal axis 111.

FIG. 9 illustrates a possible sequence for approaching an occlusive clot 40 using a large bore clot retrieval catheter 100 used in conjunction with the support frame designs disclosed herein. The clot 40 can be approached with the catheter 100 collapsed within a guide sheath 30 or other access catheter. When the vasculature 10 becomes too narrow and/or tortuous for further distal navigation with the guide sheath 30, the catheter 100 can be deployed for further independent travel distally. The catheter 100 can be highly flexible such that it is capable of navigating the M1 or other tortuous regions of the neurovascular system to reach an occlusive clot. As discussed herein, the catheter 100 may have an expanded outer diameter slightly less than that of the target vessel so the catheter is capable of distal navigation independently after deployment, or may be sized larger than the target vessel and be configured to compress radially such that it may be used to treat smaller sized vessels.

The clot retrieval catheter 100 can have a flexible elongate body 110 serving as a shaft with a large internal bore (which in some cases can be 2.032 mm (0.080 inches) or larger) and a distal tip section having a collapsible support frame of the designs disclosed herein, such as support frame 210. The large bore helps the catheter to be delivered to a target site by a variety of methods. These can include over a guidewire, over a microcatheter, with a dilator/access tool, or by itself.

In many cases, the design of the tip can be configured so that the entire catheter 100 can be delivered through (and retrieved back through) common standard 6F sheaths/8F guides, which typically have inner lumens of less than 2.286 mm (0.090 inches). The tip can self-expand once advanced to an unconstrained position distal to the distal end 32 of the guide sheath 30. As the catheter can be deployed proximal of and then be advanced independently to a remote occlusion, the support frame of the tip is designed to be able to resist collapse from the forces of aspiration, have excellent lateral flexibility in both the expanded and collapsed states, and an atraumatic profile to prevent snagging on bifurcations in vessels.

FIGS. 10A-10C illustrate example treatment steps for using a clot retrieval catheter expandable support frame of the designs disclosed herein in conjunction with a CSB catheter, as described above. While FIGS. 10A-10C illustrate the use of support frame 210, other support frames described herein (e.g., 310, 410, 510, 610, 710) may be used in the same or similar series of treatment steps. FIG. 10A illustrates support frame 210 inside a catheter shaft 110, and inside distal end 32 of guide sheath 30, as the catheter tip is moved through the target vessel 12 toward clot 40 (FIG. 10B). As shown, when inside the guide sheath 30 in its collapsed delivery configuration, support frame 210 includes collapsed inner diameter 215.

FIG. 10B illustrates support frame 210 transitioning to its expanded deployed configuration as it exits the distal end 32 of the guide sheath 30. As described above, when used in conjunction with a CSB catheter, support frame 210 can be heat set such that upon exiting the distal end 32 of the guide sheath 30, support frame 210 can expand to an expanded inner diameter 224, which is greater than the collapsed inner diameter 215. The funnel profile 816 of support frame 210 can also be symmetrical about the longitudinal axis 111, as described above with respect to FIG. 7. Even when in its expanded deployed configuration, however, the maximum outer diameter of support frame 210 can be less than an inner diameter 13 of target vessel 12 such that support frame 210 can advance distally toward clot 40 independently from and without sealing to the vessel 12.

FIG. 10C illustrates a mouth 213 of support frame 210 engulfing a proximal portion of clot 40. As shown and discussed above, support frame 210 can shorten longitudinally when placed in compression against and/or around clot 40.

FIGS. 11A-11C illustrate example treatment steps for using a clot retrieval catheter expandable support frame of the designs disclosed herein in conjunction with an LST catheter, as described above. While FIGS. 11A-11C illustrate the use of support frame 210, other support frames described herein (e.g., 310, 410, 510, 610, 710) may be used in the same or similar series of treatment steps. FIG. 11A illustrates support frame 210 inside a catheter shaft 110, and inside distal end 32 of guide sheath 30, as the catheter tip is moved through the target vessel 12 toward clot 40 (FIG. 11B). As shown, when inside the guide sheath 30 in its collapsed delivery configuration, support frame 210 includes collapsed inner diameter 215.

FIG. 11B illustrates support frame 210 exiting the distal end 32 of the guide sheath 30. As described above, when used in conjunction with an LST catheter, support frame 210 can maintain its collapsed inner diameter 215 when exiting the distal end 32 of the guide sheath 30. As shown, the distal tip section 810 of support frame 210 begins to align with a beveled mouth plane 821.

FIG. 11C illustrates a mouth 213 of support frame 210 engulfing a proximal portion of clot 40. As shown and discussed above, support frame 210 can shorten longitudinally when placed in compression against and/or around clot 40. Additionally, the funnel profile 816 can be radially offset from the longitudinal axis 111, as described above with respect to FIG. 8. And similar to when support frame 210 is used in conjunction with a CSB catheter (FIGS. 10A-10C), even when in its expanded deployed configuration, the maximum outer diameter of support frame 210 can be less than an inner diameter 13 of target vessel 12 such that support frame 210 can advance distally toward clot 40 independently from and without sealing to the vessel 12.

The invention is not necessarily limited to the examples described, which can be varied in construction and detail. The terms "distal" and "proximal" are used throughout the preceding description and are meant to refer to positions and directions relative to a treating physician. As such, "distal" or distally" refer to a position distant to or a direction away from the physician. Similarly, "proximal" or "proximally" refer to a position near or a direction towards the physician. Furthermore, the singular forms "a," "an," and "the" include plural referents unless the context clearly dictates otherwise.

As used herein, the terms "about" or "approximately" for any numerical values or ranges indicate a suitable dimensional tolerance that allows the part or collection of components to function for its intended purpose as described herein. More specifically, "about" or "approximately" may refer to the range of values ±20% of the recited value, e.g. "about 90%" may refer to the range of values from 71% to 99%.

In describing example embodiments, terminology has been resorted to for the sake of clarity. As a result, not all possible combinations have been listed, and such variants are often apparent to those of skill in the art. It is intended that each term contemplates its broadest meaning as understood by those skilled in the pertinent art. It is also to be understood that the mention of one or more steps of a method does not preclude the presence of additional method steps or intervening method steps between those steps expressly identified. Similarly, some steps of a method can be performed in a different order than those described herein without departing from the scope of the disclosed technology.

## Claims

1. A catheter tip comprising:
a. a support frame (210, 310, 410) comprising a longitudinal axis (111), a collapsed delivery configuration, an expanded deployed configuration, and a plurality of interconnected struts (218, 318, 418) defining an axial series of expansion cells (221, 321, 421); the interconnected struts joined at opposing pairs of x-connectors (217, 317, 417) spaced 180 degrees apart about the longitudinal axis;
b. each opposing pair of x-connectors rotated 90 degrees about the longitudinal axis with respect to an adjacent opposing pair of x-connectors; and wherein
c. in the collapsed delivery configuration, the support frame further comprises a collapsed inner diameter (215); and
when the support frame is placed in compression, the support frame moves to the expanded deployed configuration in which the support frame comprises a larger expanded inner diameter than in the collapsed delivery configuration and an axial length which is less than in the collapsed delivery configuration.

2. The catheter tip of claim 1, the support frame further comprising a proximal collar (115) at a proximal end (112) of the support frame; and/or, the interconnected struts comprising a curvilinear profile.

3. The catheter tip of claim 1, the support frame expanding from the collapsed inner diameter to the expanded inner diameter when impinged by an ingested clot (40)

4. The catheter tip of claim 1, the support frame further comprising an axial length (225, 325, 425), the axial length being less in the expanded deployed configuration than in the collapsed delivery configuration.

5. The catheter tip of claim 1, the series of expansion cells shortening longitudinally when the support frame is placed in compression.

6. The catheter tip of claim 1, the axial series of expansion cells comprising opposing pairs of cells spaced 180 degrees apart about the longitudinal axis; and
each opposing pair of cells rotated 90 degrees about the longitudinal axis with respect to an adjacent pair of opposing cells.

7. The catheter tip of claim 1, further comprising an offset mouth strut (220, 320, 420) at a distal end (114) of the support frame, at least a portion of the offset mouth strut residing in a plane forming an acute angle (222, 322, 422) with respect to the longitudinal axis.

8. The catheter tip of claim 1, wherein in the collapsed delivery configuration, a distal end (114) of the support frame further comprises a substantially circular cross section with a center substantially coincident with the longitudinal axis.

9. A catheter tip comprising:
a support frame (510, 610, 710) comprising a longitudinal axis (111), a collapsed delivery configuration, an expanded deployed configuration, a proximal collar at its proximal end and an axial series of hoop ribs extending in planes offset from the longitudinal axis;
the hoop ribs comprising a curvilinear profile, a non-planar cross section, and a distally unconnected peak (518, 618, 718), wherein at least one hoop rib is directly connected to the proximal collar at a connection point (519, 619, 719); wherein
in the collapsed delivery configuration, the support frame (510, 601, 710) further comprises a collapsed inner diameter; and
when the support frame is placed in compression, the support frame moves to the expanded deployed configuration in which a mouth of the support frame has a larger expanded inner diameter than in the collapsed delivery configuration and the support frame (510, 610, 710) has an axial length which is less than in the collapsed delivery configuration.

10. The catheter tip of claim 1 or claim 9, the support frame further comprising a shape memory alloy with an Austenite finish temperature less than approximately 30 degrees Celsius,
optionally wherein the support frame is heat set to have the expanded inner diameter greater than an inner diameter of the collapsed delivery configuration.

11. The catheter tip of claim 9, the distally unconnected peak of each of the hoop ribs moving proximally when the support frame is in compression during clot (40) ingestion.

12. The catheter tip of claim 9, at least a portion of each of the hoop ribs residing in a plane forming an acute angle (522, 622, 722) with respect to the longitudinal axis.

13. The catheter tip of claim 9, the support frame further comprising a larger expanded inner diameter when impinged radially by an ingested clot in the expanded deployed configuration and a smaller delivery inner diameter in the collapsed delivery configuration.

14. The catheter tip of claim 9, a distal end of the support frame in the expanded deployed configuration having a circular profile comprising a center radially offset from the longitudinal axis.

15. The catheter tip of claim 9, the support frame further comprising one or more connector ribs (620, 720) extending from a ring member (116) connected to a proximal end (112) of the support frame, the connector ribs diverging from the ring member in an offset plane at an angle to the offset plane of the hoop ribs.

## Patentansprüche

1. Katheterspitze, umfassend:
a. einen Stützrahmen (210, 310, 410), umfassend eine Längsachse (111), eine zusammengeklappte Zuführkonfiguration, eine expandierte entfaltete Konfiguration und eine Vielzahl von miteinander verbundenen Streben (218, 318, 418), die eine axiale Reihe von Expansionszellen (221, 321, 421) definieren; wobei die miteinander verbundenen Streben an gegenüberliegenden Paaren von X-Verbindern (217, 317, 417) verbunden sind, welche um 180 Grad um die Längsachse voneinander beabstandet sind;
b. jedes gegenüberliegende Paar von X-Verbindern in Bezug auf ein benachbartes gegenüberliegendes Paar von X-Verbindern um 90 Grad um die Längsachse gedreht ist; und wobei
c. in der zusammengeklappten Zuführkonfiguration der Stützrahmen ferner einen zusammengeklappten Innendurchmesser (215) umfasst; und wenn der Stützrahmen komprimiert wird, sich der Stützrahmen in die expandierte entfaltete Konfiguration bewegt, in der der Stützrahmen einen größeren expandierten Innendurchmesser als in der zusammengeklappten Zuführkonfiguration und eine axiale Länge umfasst, die geringer ist als in der zusammengeklappten Zuführkonfiguration.

2. Katheterspitze nach Anspruch 1, wobei der Stützrahmen ferner einen proximalen Kragen (115) an einem proximalen Ende (112) des Stützrahmens umfasst; und/oder die miteinander verbundenen Streben ein krummliniges Profil umfassen.

3. Katheterspitze nach Anspruch 1, wobei der Stützrahmen von dem zusammengefalteten Innendurchmesser auf den expandierten Innendurchmesser expandiert, wenn er durch ein aufgenommenes Gerinnsel (40) beaufschlagt wird.

4. Katheterspitze nach Anspruch 1, wobei der Stützrahmen ferner eine axiale Länge (225, 325, 425) umfasst, wobei die axiale Länge in der expandierten entfalteten Konfiguration geringer als in der zusammengeklappten Zuführkonfiguration ist.

5. Katheterspitze nach Anspruch 1, wobei sich die Reihe von Expansionszellen in Längsrichtung verkürzt, wenn der Stützrahmen komprimiert wird.

6. Katheterspitze nach Anspruch 1, wobei die axiale Reihe von Expansionszellen gegenüberliegende Zellpaare umfasst, die um 180 Grad um die Längsachse voneinander beabstandet sind; und
jedes gegenüberliegende Paar von Zellen in Bezug auf ein benachbartes Paar gegenüberliegender Zellen um 90 Grad um die Längsachse gedreht ist.

7. Katheterspitze nach Anspruch 1, ferner umfassend eine versetzte Mündungsstrebe (220, 320, 420) an einem distalen Ende (114) des Stützrahmens, wobei mindestens ein Abschnitt der versetzten Mündungsstrebe in einer Ebene liegt, die einen spitzen Winkel (222, 322, 422) in Bezug auf die Längsachse bildet.

8. Katheterspitze nach Anspruch 1, wobei in der zusammengeklappten Zuführkonfiguration ein distales Ende (114) des Stützrahmens ferner einen im Wesentlichen kreisförmigen Querschnitt mit einem Mittelpunkt umfasst, der im Wesentlichen mit der Längsachse zusammenfällt.

9. Katheterspitze, umfassend:
einen Stützrahmen (510, 610, 710), umfassend eine Längsachse (111), eine zusammengeklappte Zuführkonfiguration, eine expandierte entfaltete Konfiguration, einen proximalen Kragen an seinem proximalen Ende und eine axiale Reihe von Ringrippen, die sich in von der Längsachse versetzten Ebenen erstrecken;
die Ringrippen, die ein krummliniges Profil umfassen, einen nicht-planaren Querschnitt und eine distal unverbundene Spitze (518, 618, 718), wobei mindestens eine Ringrippe an einer Verbindungsstelle (519, 619, 719) direkt mit dem proximalen Kragen verbunden ist; wobei
in der zusammengeklappten Zuführkonfiguration der Stützrahmen (510, 601, 710) ferner einen zusammengeklappten Innendurchmesser umfasst; und
wenn der Stützrahmen komprimiert wird, sich der Stützrahmen in die expandierte entfaltete Konfiguration bewegt, in der eine Mündung des Stützrahmens einen größeren expandierten Innendurchmesser aufweist als in der zusammengeklappten Zuführkonfiguration und der Stützrahmen (510, 610, 710) eine axiale Länge aufweist, die geringer ist als in der zusammengeklappten Zuführkonfiguration.

10. Katheterspitze nach Anspruch 1 oder 9, wobei der Stützrahmen ferner eine Formgedächtnislegierung mit einer Austenit-Endtemperatur von weniger als etwa 30 Grad Celsius umfasst,
optional wobei der Stützrahmen thermofixiert ist, um einen expandierten Innendurchmesser aufzuweisen, der größer als ein Innendurchmesser der zusammengeklappten Zuführkonfiguration ist.

11. Katheterspitze nach Anspruch 9, wobei sich die distal unverbundene Spitze jeder der Ringrippen proximal bewegt, wenn der Stützrahmen während der Aufnahme des Gerinnsels (40) komprimiert wird.

12. Katheterspitze nach Anspruch 9, wobei mindestens ein Abschnitt jeder der Ringrippen in einer Ebene liegt, die einen spitzen Winkel (522, 622, 722) in Bezug auf die Längsachse bildet.

13. Katheterspitze nach Anspruch 9, wobei der Stützrahmen ferner einen größeren expandierten Innendurchmesser bei radialer Beaufschlagung durch ein aufgenommenes Gerinnsel in der expandierten entfalteten Konfiguration und einen kleineren Zuführinnendurchmesser in der zusammengeklappten Zuführkonfiguration aufweist.

14. Katheterspitze nach Anspruch 9, wobei ein distales Ende des Stützrahmens in der expandierten entfalteten Konfiguration ein kreisförmiges Profil aufweist, das einen radial von der Längsachse versetzten Mittelpunkt umfasst.

15. Katheterspitze nach Anspruch 9, wobei der Stützrahmen ferner eine oder mehrere Verbindungsrippen (620, 720) umfasst, die sich von einem Ringelement (116) erstrecken, das mit einem proximalen Ende (112) des Stützrahmens verbunden ist, wobei die Verbindungsrippen von dem Ringelement in einer versetzten Ebene in einem Winkel zu der versetzten Ebene der Reifenrippen divergieren.

## Revendications

1. Pointe de cathéter comprenant :
a. un châssis de support (210, 310, 410) comprenant un axe longitudinal (111), une configuration de pose rétractée, une configuration déployée expansée, et une pluralité d'entretoises interreliées (218, 318, 418) définissant une série axiale de cellules d'expansion (221, 321, 421) ; les entretoises interreliées étant jointes au niveau de paires opposées d'éléments de liaison en x (217, 317, 417) espacées de 180 degrés autour de l'axe longitudinal ;
b. chaque paire opposée d'éléments de liaison en x étant mise en rotation à 90 degrés autour de l'axe longitudinal par rapport à une paire opposée adjacente d'éléments de liaison en x ; et dans laquelle
c. dans la configuration de pose rétractée, le châssis de support comprend en outre un diamètre interne rétracté (215) ; et lorsque le châssis de support est placé en compression, le châssis de support passe à la configuration déployée expansée dans laquelle le châssis de support comprend un diamètre interne expansé plus grand que dans la configuration de pose rétractée et une longueur axiale qui est plus petite que dans la configuration de pose rétractée.

2. Pointe de cathéter selon la revendication 1, le châssis de support comprenant en outre un collier proximal (115) au niveau d'une extrémité proximale (112) du châssis de support ; et/ou, les entretoises interreliées comprenant un profil curviligne.

3. Pointe de cathéter selon la revendication 1, le châssis de support s'expansant du diamètre interne rétracté au diamètre interne expansé lorsqu'il est heurté par un caillot (40) ingéré

4. Pointe de cathéter selon la revendication 1, le châssis de support comprenant en outre une longueur axiale (225, 325, 425), la longueur axiale étant plus petite dans la configuration déployée expansée que dans la configuration de pose rétractée.

5. Pointe de cathéter selon la revendication 1, la série de cellules d'expansion se raccourcissant longitudinalement lorsque le châssis de support est placé en compression.

6. Pointe de cathéter selon la revendication 1, la série axiale de cellules d'expansion comprenant des paires opposées de cellules espacées de 180 degrés autour de l'axe longitudinal ; et
chaque paire opposée de cellules étant mise en rotation à 90 degrés autour de l'axe longitudinal par rapport à une paire adjacente de cellules opposées.

7. Pointe de cathéter selon la revendication 1, comprenant en outre une entretoise d'embouchure décalée (220, 320, 420) au niveau d'une extrémité distale (114) du châssis de support, au moins une partie de l'entretoise d'embouchure décalée se trouvant dans un plan formant un angle aigu (222, 322, 422) par rapport à l'axe longitudinal.

8. Pointe de cathéter selon la revendication 1, dans laquelle dans la configuration de pose rétractée, une extrémité distale (114) du châssis de support comprend en outre une coupe transversale sensiblement circulaire avec un centre coïncidant sensiblement avec l'axe longitudinal.

9. Pointe de cathéter comprenant :
un châssis de support (510, 610, 710) comprenant un axe longitudinal (111), une configuration de pose rétractée, une configuration déployée expansée, un collier proximal au niveau de son extrémité proximale et une série axiale de baleines de cerclage s'étendant dans des plans décalés par rapport à l'axe longitudinal ;
les baleines de cerclage comprenant un profil curviligne, une coupe transversale non plane et un pic non relié distalement (518, 618, 718), dans laquelle au moins une baleine de cerclage est reliée directement au collier proximal au niveau d'un point de liaison (519, 619, 719) ; dans laquelle
dans la configuration de pose rétractée, le châssis de support (510, 601, 710) comprend en outre un diamètre interne rétracté ; et
lorsque le châssis de support est placé en compression, le châssis de support passe à la configuration déployée expansée dans laquelle une embouchure du châssis de support a un diamètre interne expansé plus grand que dans la configuration de pose rétractée et le châssis de support (510, 610, 710) a une longueur axiale qui est plus petite que dans la configuration de pose rétractée.

10. Pointe de cathéter selon la revendication 1 ou la revendication 9, le châssis de support comprenant en outre un alliage à mémoire de forme avec température de finition austénitique inférieure à approximativement 30 degrés Celsius,
facultativement dans laquelle le châssis de support est thermofixé pour avoir le diamètre interne expansé plus grand qu'un diamètre interne de la configuration de pose rétractée.

11. Pointe de cathéter selon la revendication 9, le pic non relié distalement de chacune des baleines de cerclage se déplaçant proximalement lorsque le châssis de support en compression pendant une ingestion de caillot (40).

12. Pointe de cathéter selon la revendication 9, au moins une partie de chacune des baleines de cerclage se trouvant dans un plan formant un angle aigu (522, 622, 722) par rapport à l'axe longitudinal.

13. Pointe de cathéter selon la revendication 9, le châssis de support comprenant en outre un diamètre interne expansé plus grand lorsqu'il est heurté radialement par un caillot ingéré dans la configuration déployée expansée et un diamètre interne de pose plus petit dans la configuration de pose rétractée.

14. Pointe de cathéter selon la revendication 9, une extrémité distale du châssis de support dans la configuration déployée expansée ayant un profil circulaire comprenant un centre radialement décalé par rapport à l'axe longitudinal.

15. Pointe de cathéter selon la revendication 9, le châssis de support comprenant en outre une ou plusieurs baleines de liaison (620, 720) s'étendant à partir d'un élément annulaire (116) relié à une extrémité proximale (112) du châssis de support, les baleines de liaison divergeant de l'élément annulaire dans un plan de décalage selon un angle par rapport au plan de décalage des baleines de cerclage.
